# EUROPEAN PATENT APPLICATION

(11) **EP 3 819 911 A2**
(43) Date of publication of application: **12.05.2021**
(21) Application number: 19846767.2
(22) Date of filing: 07.08.2019
(51) Int. Cl.: G16H 50/20

(54) **BIOIMAGE DIAGNOSIS SYSTEM, BIOIMAGE DIAGNOSIS METHOD, AND TERMINAL FOR EXECUTING SAME**

(30) Priority: 08.08.2018 KR 20180092648
(71) Applicant: Deep Bio Inc., Seoul 08394 (KR)
(72) Inventor: KIM, Sun Woo, Seongnam-si Gyeonggi-do 13599 (KR)
(74) Representative: Flügel Preissner Schober Seidel
(86) International application number: PCT/KR2019/009848
(87) International publication number: WO 2020/032562

(57) **Abstract**

Disclosed are a biometric image diagnosis system and method for aiding a diagnostician to refer to an existing diagnostic history and form an accurate diagnosis of a disease from the image of a biological tissue. According to one aspect of the present invention, the biometric image diagnosis system comprises: a first search module configured to retrieve at least one similar biometric image having similar characteristics to a predetermined biometric image to be diagnosed, from a DB storing a plurality of pre-diagnosed biometric images and diagnostic results with regards to the plurality of pre-diagnosed biometric images; a second search module configured to retrieve at least one pre-diagnosed biometric image pre-diagnosed by a predetermined diagnostician from the DB; a communications module configured to transmit the at least one similar biometric image and the at least one pre-diagnosed biometric image to a terminal of the diagnostician; and a storage module configured to store in the DB, a disease diagnosis result with respect to the biometric image to be diagnosed, received from the terminal of the diagnostician, wherein the terminal of the diagnostician is configured to display the biometric image to be diagnosed, the at least one similar biometric image, and the at least one pre-diagnosed biometric image.

## Description

### TECHNICAL FIELD

The present invention relates to a biometric image diagnosis system, a biometric image diagnosis method, and a terminal for executing the same. More specifically, the present invention relates to a biometric image diagnosis system, a biometric image diagnosis method, and a terminal for executing the same, which support a diagnostician to accurately diagnose a disease from an image of a biological tissue with reference to an existing diagnosis history.

### BACKGROUND ART

One of major tasks performed by pathology or a pathology department is to read a slide, which is a biometric image of a patient, or a part thereof (e.g., a tile, a patch, etc.) and perform diagnosis for determining a state or symptom of a specific disease. The diagnosis like this is a method that depends on the experience and knowledge of medical personnel skilled for an extended period of time. Generally, in a method of diagnosing a disease using a biometric image, a medical professional skilled in the biometric image annotates a state of a specific disease (e.g., whether or not cancer is expressed).

However, due to the characteristics of determining by experience, the criteria of determination may be slightly vary depending on the timing of determination even for experienced medical personnel, and it is much probable that slightly different determination criteria may be applied according to the diagnostician. Accordingly, it is urgently required to provide a means for improving consistency in diagnosing a disease through a biometric image.

### DISCLOSURE OF INVENTION

### TECHNICAL PROBLEM

Therefore, the present invention has been made in view of the above problems, and it is an object of the present invention to provide a technical spirit that allows a diagnostician to accurately diagnose a disease from an image of a biological tissue with reference to an existing diagnosis history.

### TECHNICAL SOLUTION

To accomplish the above object, according to one aspect of the present invention, there is provided a biometric image diagnosis system comprising: a first search module for searching for at least one similar biometric image having characteristics similar to those of a predetermined diagnosis target biometric image from a DB that stores a plurality of previously diagnosed biometric images and diagnosis results of the plurality of previously diagnosed biometric images; a second search module for searching for at least one previously diagnosed biometric image previously diagnosed by a predetermined diagnostician from the DB; a communication module for transmitting the at least one similar biometric image and the at least one previously diagnosed biometric image to a terminal of the diagnostician; and a storage module for storing a disease diagnosis result of the diagnosis target biometric image received from the terminal of the diagnostician in the DB, wherein the terminal of the diagnostician displays the diagnosis target biometric image, the at least one similar biometric image, and the at least one previously diagnosed biometric image.

In an embodiment, the second search module may search for an image having characteristics similar to those of the diagnosis target biometric image among the biometric images previously diagnosed by the diagnostician from the DB.

In an embodiment, the terminal of the diagnostician may display the diagnosis target biometric image in a first region of a diagnosis UI including the first region, a second region, and a third region, display the at least one similar biometric image in the second region, and display the at least one previously diagnosed biometric image in the third region.

In an embodiment, the diagnosis UI may further include a fourth region, and the terminal of the diagnostician may display an image enlarging the diagnosis target biometric image, any one among the at least one similar biometric image, or any one among the at least one previously diagnosed biometric image in the fourth region.

In an embodiment, the disease may be prostate cancer.

According to another aspect of the present invention, there is provided a biometric image diagnosis system comprising: a search module for searching for at least one previously diagnosed biometric image having characteristics similar to those of a predetermined diagnosis target biometric image among biometric images previously diagnosed by a predetermined diagnostician from a DB that stores a plurality of previously diagnosed biometric images and diagnosis results of the plurality of previously diagnosed biometric images; a communication module for transmitting the at least one similar biometric image and the at least one previously diagnosed biometric image to a terminal of the diagnostician; and a storage module for storing a disease diagnosis result of the diagnosis target biometric image received from the terminal of the diagnostician in the DB, wherein the terminal of the diagnostician displays the diagnosis target biometric image and the at least one previously diagnosed biometric image.

According to another aspect of the present invention, there is provided a terminal allowing a diagnostician to diagnose a disease through a biometric image, the terminal comprising: a reception module for receiving at least one similar biometric image having characteristics similar to those of a predetermined diagnosis target biometric image, and at least one previously diagnosed biometric image previously diagnosed by the diagnostician from a biometric image diagnosis system; a display module for displaying the diagnosis target biometric image, the at least one similar biometric image, and the at least one previously diagnosed biometric image; and an annotation module for annotating a disease diagnosis result of the diagnosis target biometric image on the diagnosis target biometric image, wherein the display module displays the diagnosis target biometric image in a first region of a diagnosis UI including the first region, a second region, and a third region, displays the at least one similar biometric image in the second region, and displays the at least one previously diagnosed biometric image in the third region.

According to another aspect of the present invention, there is provided a biometric image diagnosis method comprising the steps of: searching for at least one similar biometric image having characteristics similar to those of a predetermined diagnosis target biometric image from a DB that stores a plurality of previously diagnosed biometric images and diagnosis results of the plurality of previously diagnosed biometric images, by a biometric image diagnosis system; searching for at least one previously diagnosed biometric image previously diagnosed by a predetermined diagnostician from the DB, by the biometric image diagnosis system; transmitting the at least one similar biometric image and the at least one previously diagnosed biometric image to a terminal of the diagnostician, by the biometric image diagnosis system; and storing a disease diagnosis result of the diagnosis target biometric image received from the terminal of the diagnostician in the DB, by the biometric image diagnosis system, wherein the terminal of the diagnostician displays the diagnosis target biometric image, the at least one similar biometric image, and the at least one previously diagnosed biometric image.

In an embodiment, the step of searching for at least one previously diagnosed biometric image previously diagnosed by the diagnostician from the DB may include the step of searching for an image having characteristics similar to those of the diagnosis target biometric image among the biometric images previously diagnosed by a user from the DB.

According to another aspect of the present invention, there is provided a biometric image diagnosis method comprising the steps of: searching for at least one previously diagnosed biometric image having characteristics similar to those of a diagnosis target biometric image among biometric images previously diagnosed by a predetermined diagnostician from a DB that stores a plurality of previously diagnosed biometric images and diagnosis results of the plurality of previously diagnosed biometric images, by a biometric image diagnosis system; transmitting the at least one previously diagnosed biometric image to a terminal of the diagnostician, by the biometric image diagnosis system; and storing a disease diagnosis result of the diagnosis target biometric image received from the terminal of the diagnostician in the DB, by the biometric image diagnosis system, wherein the terminal of the diagnostician displays the diagnosis target biometric image and the at least one previously diagnosed biometric image.

According to another aspect of the present invention, there is provided a biometric image diagnosis method executed in a terminal allowing a diagnostician to diagnose a disease through a biometric image, the method comprising the steps of: receiving at least one similar biometric image having characteristics similar to those of a predetermined diagnosis target biometric image, and at least one previously diagnosed biometric image previously diagnosed by the diagnostician from a biometric image diagnosis system, by the terminal; displaying the diagnosis target biometric image, the at least one similar biometric image, and the at least one previously diagnosed biometric image, by the terminal; and annotating a disease diagnosis result of the diagnosis target biometric image on the diagnosis target biometric image, wherein the display step includes the steps of: displaying the diagnosis target biometric image in a first region of a diagnosis UI including the first region, a second region, and a third region, displaying the at least one similar biometric image in the second region, and displaying the at least one previously diagnosed biometric image in the third region.

According to another aspect of the present invention, there is provided a computer program installed in a data processing device and recorded in a medium for performing the method described above.

### ADVANTAGEOUS EFFECTS

According to the spirit of the present invention, there is an effect of allowing a diagnostician to accurately diagnose a disease from an image of a biological tissue with reference to an existing diagnosis history. When a disease is diagnosed through a biometric image, frequently, it is difficult for a diagnostician to maintain consistency in the diagnosis criteria as time varies, and according to the spirit of the present invention, it may be very effective for the diagnostician to derive a consistent diagnosis result.

### BRIEF DESCRIPTION OF THE DRAWINGS

To more sufficiently understand the drawings cited in the detailed description of the present invention, a brief description of each drawing is provided.
FIG. 1 is a view showing an operating environment of a biometric image diagnosis method according to the spirit of the present invention.
FIG. 2 is a view showing the schematic configuration of a biometric image diagnosis system 100 according to an embodiment of the present invention.
FIG. 3 is a block diagram schematically showing the configuration of a terminal of the diagnostician 20 according to an embodiment of the present invention.
FIG. 4 is a view showing an example of a diagnosis UI 300 displayed by a display module 22.
FIG. 5 is a flowchart illustrating a schematic process of a biometric image diagnosis method according to an embodiment of the present invention

### BEST MODE FOR CARRYING OUT THE INVENTION

As the present invention may apply various modifications and have various embodiments, specific embodiments will be illustrated in the drawings and described in detail in the detailed description. However, this is not intended to limit the present invention to the specific embodiments, and it should be understood to include all modifications, equivalents, and substitutes included in the spirit and scope of the present invention. In describing the present invention, when it is determined that detailed description of a related known technology may obscure the subject matter of the present invention, the detailed description will be omitted.

Although the terms such as first, second and the like may be used to describe various components, the components should not be limited by the terms. These terms are only used for the purpose of distinguishing one component from the other components.

The terms used in the present application are only used to describe specific embodiments, and are not intended to limit the present invention. Singular expressions include plural expressions unless the context clearly indicates otherwise.

In this specification, the terms such as "comprise" or "have" are intended to designate the presence of features, numbers, steps, actions, components, parts, or combinations thereof described in the specification, and it should be understood that they do not preclude the possibility of the presence or addition of one or more other features or numbers, steps, actions, components, parts, or combinations thereof.

In addition, in this specification, when any one component 'transmits' data to another component, this means that the component may directly transmit the data to another component, or may transmit the data to another component through at least one other component. On the contrary, when one component 'directly transmits' data to another component, it means that the data is transmitted from the component to another component without passing through the other components.

Hereinafter, the present invention will be described in detail focusing on the embodiments of the present invention with reference to the accompanying drawings. The same reference numerals in each drawing indicate the same members.

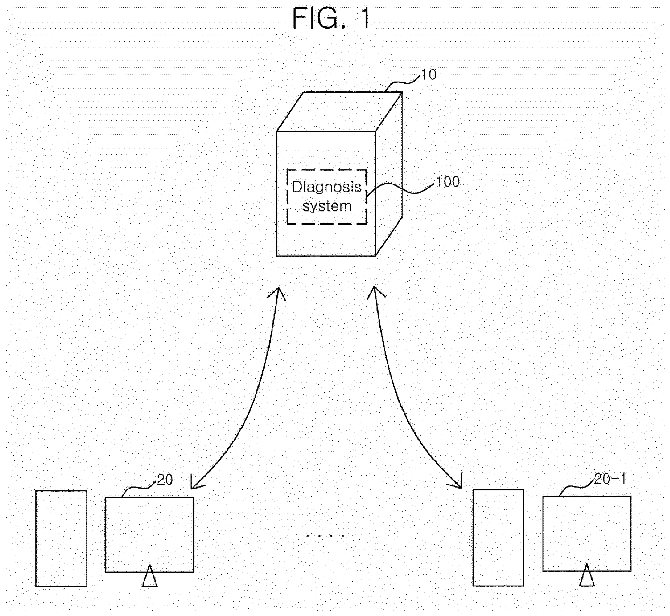

FIG. 1 is a view showing an operating environment of a biometric image diagnosis method according to the spirit of the present invention. Referring to FIG. 1, the biometric image diagnosis method according to the spirit of the present invention may be performed by a biometric image diagnosis system 100 and terminal of the diagnosticians 20 to 20-1.

The biometric image diagnosis system 100 according to the spirit of the present invention may be installed in a predetermined server 10 to implement the spirit of the present invention. The server 10 means a data processing device having a computing ability for implementing the spirit of the present invention, and those skilled in the art may easily infer that any device capable of performing a specific service such as a personal computer, a portable terminal or the like, as well as a data processing device that can be generally accessed by a client (terminal) through a network, may be defined as a server.

The server 10 may include a processor 11 and a storage device 12. The processor may mean a computing device capable of driving a program for implementing the spirit of the present invention.

The storage device 12 may mean a data storage means capable of storing programs and various data needed for implementing the spirit of the present invention, and may be implemented as a plurality of storage means according to implementation examples. In addition, the storage device may mean a temporary storage device or a memory that may be included in the processor, as well as a main memory device or the like included in the server 10.

Although it is shown in FIG. 1 that the biometric image diagnosis system 100 is implemented as any one physical device, those skilled in the art may easily infer that the biometric image diagnosis system 100 according to the spirit of the present invention may be implemented by organically combining a plurality of physical devices as needed.

In this specification, when it is described that diagnosis is performed, it may mean that a predetermined diagnostician (e.g., a doctor, etc.) confirms a slide, which is a biometric image expressing a biological tissue, or a part thereof (e.g., a patch or a tile) and makes a determination on a specific disease. Accordingly, a diagnosis result of a biometric image may include a degree of progression (or a probability to correspond to the degree of progression) of a specific disease, as well as whether a specific disease is expressed. For example, when the spirit of the present invention is used for diagnosis of prostate cancer, the Gleason Pattern or Gleason Score, which are indexes indicating a degree of progression of prostate cancer, may be included in the diagnosis result. For example, the Gleason score has a value of 2 to 5, and a larger number indicates a higher degree of expression of the prostate cancer. Meanwhile, the diagnosis result may include a part where the disease is expressed.

A biometric image to be diagnosed (hereinafter, referred to as a 'diagnosis target biometric image') is displayed on the terminal 20, and a diagnostician may analyze the diagnosis target biometric image to diagnose a disease, and input a diagnosis result into the terminal. Then, the terminal 20 may annotate the diagnosis result on the diagnosis target biometric image. Annotation may mean generating predetermined information expressing a diagnosis result of the diagnosis target biometric image and matching the diagnosis result to a corresponding biometric image.

Meanwhile, the terminal 20 may further display an auxiliary reference biometric image that can be referred to by a diagnostician to perform diagnosis. Such an auxiliary biometric image may be provided by the biometric image diagnosis system 100. The biometric image diagnosis system 100 may search for a specific image highly related to the diagnosis target biometric image from a database (hereinafter, referred to as DB), and provide the specific image to the terminal 20.

Meanwhile, the terminal 20 may provide a diagnosis result of the diagnosis target biometric image to the biometric image diagnosis system 100, and the biometric image diagnosis system 100 may store the diagnosis target biometric image and a diagnosis result thereof in the DB.

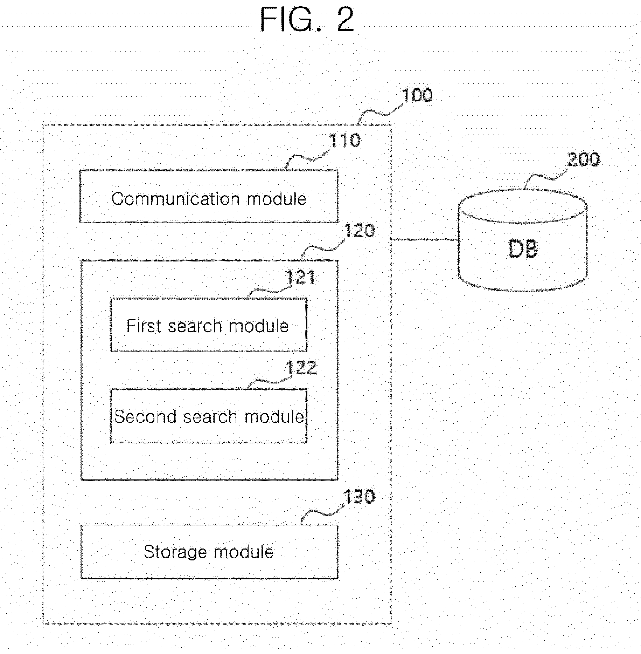

FIG. 2 is a view showing the schematic configuration of a biometric image diagnosis system 100 according to an embodiment of the present invention.

Referring to FIG. 2, the biometric image diagnosis system 100 may include a communication module 110, a search module 120, and a storage module 130. According to embodiments of the present invention, some components among the components described above may not necessarily correspond to the components essential to implementation of the present invention, and in addition, it goes without saying that the biometric image diagnosis system 100 may include more components according to embodiments. For example, the biometric image diagnosis system 100 may further include a control module (not shown) for controlling the functions and/or resources of other components (e.g., the communication module 110, the search module 120, the storage module 130, etc.) of the biometric image diagnosis system 100.

The diagnosis system 100 may mean a logical configuration provided with hardware resources and/or software needed to implement the spirit of the present invention, and does not necessarily mean a physical component or a device. That is, the diagnosis system 100 may mean a logical combination of hardware and/or software provided to implement the spirit of the present invention, and may be implemented as a set of logical components installed in devices separated from each other when needed to perform their functions to implement the spirit of the present invention. In addition, the diagnosis system 100 may mean a set of components separately implemented for each function or role for implementing the spirit of the present invention. For example, the communication module 110, the search module 120, and/or the storage module 130 may be located in different physical devices, or may be located in the same physical device. In addition, according to implementation examples, combinations of software and/or hardware configuring each of the communication module 110, the search module 120, and the storage module 130 may also be located in different physical devices, and components located in different physical devices may be organically combined with each other to implement each of the modules.

In addition, in this specification, a module may mean a functional or structural combination of hardware for performing the spirit of the present invention and software for driving the hardware. For example, those skilled in the art may easily infer that the module may mean a predetermined code and a logical unit of hardware resources for executing the predetermined code, and does not necessarily mean a physically connected code or a single type of hardware.

The communication module 110 may communicate with the terminals 20 to 20-1 through a wired/wireless communication network or a predetermined communication interface, and transmit and receive various information, data and/or signals needed for implementing the spirit of the present invention.

The search module 120 may search the DB 200 for a biometric image as a reference for diagnosing a biometric image that is an actual target of diagnosis, and the searched biometric image is transmitted to the terminal of the diagnostician 20 through the communication module 110.

The DB 200 may store a plurality of previously diagnosed biometric images and a diagnosis result of the previously diagnosed biometric images. The DB 200 may store diagnosis results of several previously diagnosed biometric images diagnosed previously. The previously diagnosed biometric images may be biometric images diagnosed by various diagnosticians. That is, when several diagnosticians perform diagnosis on several biometric images and derive diagnosis results thereof, respectively, corresponding biometric images and diagnosis results thereof may be stored in the DB 200. Accordingly, the DB 200 may further store information for identifying a diagnostician who has diagnosed a corresponding biometric image, in addition to the plurality of previously diagnosed biometric images and the diagnosis results of the plurality of previously diagnosed biometric images.

In an embodiment, the search module 120 may include a first search module 121 and a second search module 122.

The first search module 121 may search the DB 200 for at least one similar biometric image having characteristics similar to those of a diagnosis target biometric image.

In an embodiment, the first search module 121 may determine a biometric image having image features similar to those of a diagnosis target biometric image as a similar biometric image. The methods for determining whether the image features are similar may be diverse. For example, the similarity may be determined by comparing feature points of two images, and a machine such as a previously learned neural network may be used to determine whether the images are similar.

The first search module 121 may arrange several searched similar biometric images in order of similarity and output only some of them.

The second search module 122 may search for at least one previously diagnosed biometric image previously diagnosed by a predetermined diagnostician from the DB 200 previously diagnosed by a diagnostician corresponding to the terminal 20.

According to embodiments, the second search module 122 may search for only some biometric images satisfying a specific criterion among the biometric images previously diagnosed by the diagnostician. Particularly, the second search module 122 may search for only the images having characteristics similar to those of the diagnosis target biometric image among the biometric images previously diagnosed by the diagnostician. In this case, the second search module 121 may arrange several searched previously diagnosed biometric images in order of similarity and output only some of them.

Meanwhile, according to embodiments, the search module 120 may include only one among the first search module 121 and the second search module 122 described above, and may further include a separate detailed search module for searching for biometric images to be used as a reference for diagnosis in a method different from those of the search modules.

The communication module 110 may transmit the at least one similar biometric image searched by the search module 120 and/or the at least one previously diagnosed biometric image to the terminal of the diagnostician 20.

Thereafter, diagnosis on the diagnosis target biometric image may be performed on the terminal 20, and the communication module 110 may receive a result of diagnosis on the diagnosis target biometric image from the terminal 20.

Then, the storage module 130 may store a disease diagnosis result of the diagnosis target biometric image received from the terminal of the diagnostician 20 in the DB 200.

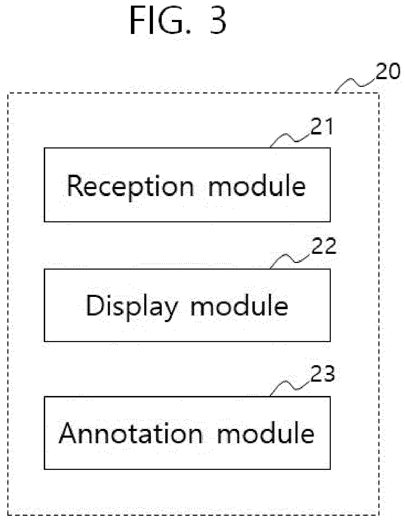

FIG. 3 is a block diagram schematically showing the configuration of a terminal of the diagnostician 20 according to an embodiment of the present invention.

Referring to FIG. 3, the terminal 20 may include a reception module 21, a display module 22, and an annotation module 23.

The reception module 21 may communicate with the biometric image diagnosis system 100 through a wired/wireless communication network or a predetermined communication interface, and transmit and receive various information, data and/or signals needed for implementing the spirit of the present invention.

For example, the reception module 21 may receive at least one similar biometric image having characteristics similar to those of the diagnosis target biometric image, and at least one previously diagnosed biometric image previously diagnosed by the diagnostician from the biometric image diagnosis system 100.

The display module 22 may display a diagnosis user interface (UI) for diagnosing the diagnosis target biometric image. A biometric image that is a reference for diagnosis, i.e., a similar biometric image and/or a previously diagnosed biometric image provided by the biometric image diagnosis system 100, as well as the diagnosis target biometric image, may be displayed on the diagnosis UI. In addition, the diagnosis UI may further include various UIs for annotating diagnosis results.

The annotation module 23 may annotate a disease diagnosis result of the diagnosis target biometric image on the diagnosis target biometric image.

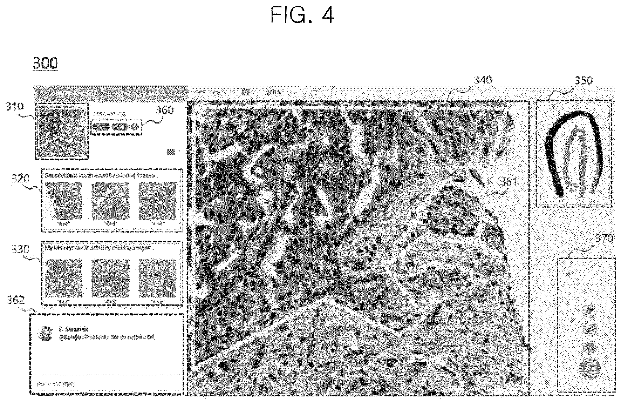

FIG. 4 is a view showing an example of a diagnosis UI 300 displayed by a display module 22.

Referring to FIG. 4, the diagnosis UI 300 includes a first region 310 for displaying a diagnosis target biometric image, a second region 320 for displaying similar biometric images, and a third region 330 for displaying previously diagnosed biometric images. A diagnostician may perform diagnosis on the diagnosis target biometric image with reference to the biometric images displayed in the second region 320 and the third region 330.

In an embodiment, when there are several similar biometric images or previously diagnosed biometric images, the display module 22 may display the images in order of similarity.

In an embodiment, previously diagnosed diagnosis results corresponding to each of the reference biometric images displayed in the second region or the third region may be further displayed.

When a disease is diagnosed through a biometric image, frequently, it is difficult for a diagnostician to maintain consistency in the diagnosis criteria as time varies, and according to the spirit of the present invention as described above, it may be very effective for the diagnostician to derive a consistent diagnosis result.

Describing in more detail, as described above, a similar biometric image is an image having characteristics similar to those of a diagnosis target biometric image among previously diagnosed biometric images stored in the DB 200. Accordingly, as the diagnostician performs diagnosis with reference to the similar biometric images displayed on the diagnosis UI 300, there is an effect of deriving a diagnosis result consistent with diagnosis results of other biological images having characteristics similar to those of the diagnosis target biometric image.

In addition, the previously diagnosed biometric image is a biometric image diagnosed in the past by a diagnostician who currently diagnoses the diagnosis target biometric image. Accordingly, as the diagnostician performs diagnosis with reference to the previously diagnosed biometric image displayed on the diagnosis UI 300, there is an effect of deriving a diagnosis result that maintains consistency.

Meanwhile, the diagnosis UI 300 may further include a fourth region 340 for displaying an enlarged image.

The enlarged image may be an image enlarging any one among the diagnosis target biometric image and the similar biometric images, or any one among the previously diagnosed biometric images. That is, a diagnostician may select any one among the diagnosis target biometric image and the similar biometric images, or any one among the previously diagnosed biometric images, and the display module 22 may display an enlarged image of the selected biometric image in the fourth region 340.

An example of enlarging a diagnosis target biometric image is shown in the example of FIG. 4. As shown in the example of FIG. 4, a diagnostician may specify a region 361 expressing a disease on the enlarged image 340 using a drawing tool 370 included in the diagnosis UI 300.

Meanwhile, the diagnosis UI 300 may include an annotation UI 360, and a diagnostician may input whether a disease is expressed or a state of the disease through the annotation UI 360.

In addition, the diagnosis UI 330 may further include a UI 362 through which a diagnostician may input comments on the diagnosis result.

Meanwhile, the diagnosis target biometric image displayed in the first region 310 may be a part of a specific slide. In this case, the entire slide may be displayed in a specific region 350 on the diagnosis UI 300, and a diagnostician may move a region to be diagnosed among the entire slide. When the movement is completed, a partial region corresponding thereto may be determined as a diagnosis target biometric image, and then diagnosis may be performed in the process described above.

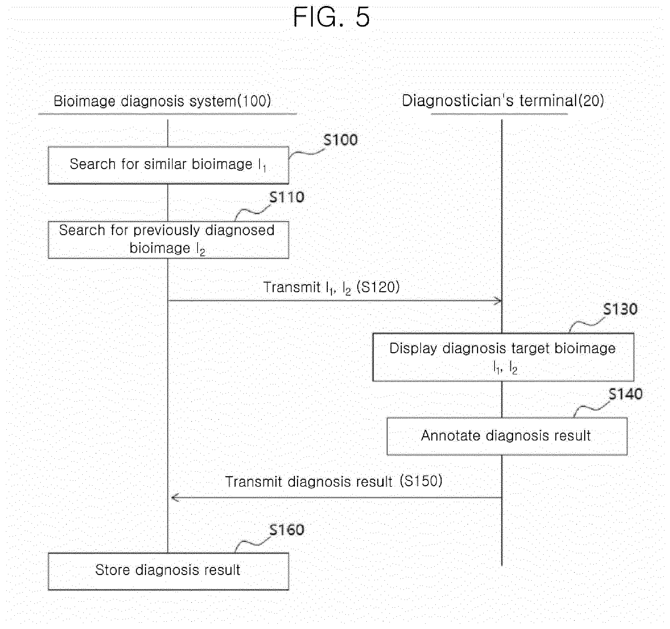

FIG. 5 is a flowchart illustrating a schematic process of a biometric image diagnosis method according to an embodiment of the present invention.

Referring to FIG. 5, the biometric image diagnosis system 100 may search for at least one similar biometric image I₁ having characteristics similar to those of the diagnosis target biometric image from the DB 200 that stores a plurality of previously diagnosed biometric images and diagnosis results of the plurality of previously diagnosed biometric images (S100).

In addition, the biometric image diagnosis system 100 may search for at least one previously diagnosed biometric image I₂ previously diagnosed by a diagnostician from the DB 200 (S110).

The searched similar biometric image and previously diagnosed biometric image may be transmitted to the terminal of the diagnostician 20 (S120).

The diagnostician's terminal 20 may display the diagnosis UI 300 showing a diagnosis target biometric image, similar biometric images, and previously diagnosed biometric images (S130), and the diagnostician may perform diagnosis on the diagnosis target biometric image with reference to the similar biometric images and the previously diagnosed biometric images, and input a diagnosis result.

Then, the diagnostician's terminal 20 may annotate the disease diagnosis result of the diagnosis target biometric image on the diagnosis target biometric image (S140) and transmit the diagnosis result to the biometric image diagnosis system 100.

Then, the biometric image diagnosis system 100 may store the received diagnosis result in the DB 200 together with the diagnosis target biometric image.

In addition, although an example of applying the spirit of the present invention to prostate cancer has been mainly described in this specification, those skilled in the art may easily infer that accurate diagnosis and visualization of a diagnosis result are possible when the spirit of the present invention is applied to other cancer or diseases that need diagnosis to be performed on a specific tissue considering the state of surrounding tissues of the tissue, as well as the specific tissue.

Meanwhile, the biometric image diagnosis method according to the spirit of the present invention may be used to more effectively produce training data that are used to train a machine for diagnosing a disease using a biometric image.

Meanwhile, according to implementation examples, the biometric image diagnosis system 100 and/or the terminal 20 may include a processor and a memory for storing programs executed by the processor. The processor may include single-core CPUs or multi-core CPUs. The memory may include high-speed random-access memory and may include one or more non-volatile memory devices such as magnetic disk storage devices, flash memory devices, and other non-volatile solid state memory devices. Access to the memory by the processor and other components may be controlled by a memory controller.

Meanwhile, the biometric image diagnosis method according to an embodiment of the present invention may be implemented in the form of a computer-readable program command and stored in a computer-readable recording medium, and control programs and target programs according to an embodiment of the present invention may also be stored in the computer-readable recording medium. The computer-readable recording medium includes all types of recording devices for storing data that can be read by a computer system.

The program commands recorded in the recording medium may be specially designed and configured for the present invention, or may be known to and used by those skilled in the software field.

Examples of the computer-readable recording medium include magnetic media such as hard disks, floppy disks, and magnetic tapes, optical media such as CD-ROMs and DVDs, magnetooptical media such as floptical disks, and hardware devices specially configured to store and execute program commands, such as ROM, RAM, flash memory and the like. In addition, the computer-readable recording medium may be distributed in computer systems connected through a network to store and execute computer-readable codes in a distributed manner.

Examples of program instructions include high-level language codes that can be executed by a device that electronically processes information using an interpreter or the like, e.g., a computer, as well as machine language codes such as those produced by a compiler.

The hardware device described above may be configured to execute as one or more software modules to perform the operation of the present invention, and vice versa.

The above description of the present invention is for illustrative purposes, and those skilled in the art may understand that it is possible to easily transform into other specific forms without changing the spirit or essential features of the present invention. Therefore, it should be understood that the embodiments described above are illustrative and nonlimiting in all respects. For example, each component described as a single form may be implemented in a distributed manner, and in the same manner, components described as being distributed may also be implemented in a combined form.

The scope of the present invention is indicated by the claims described below rather than the detailed description, and the meaning and scope of the claims and all changes or modified forms derived from the equivalent concepts thereof should be interpreted as being included in the scope of the present invention.

### INDUSTRIAL APPLICABILITY

The present invention may be used in "a biometric image diagnosis system, a biometric image diagnosis method, and a terminal for executing the same".

## Claims

1. A biometric image diagnosis system comprising:
a first search module for searching for at least one similar biometric image having characteristics similar to those of a predetermined diagnosis target biometric image from a DB that stores a plurality of previously diagnosed biometric images and diagnosis results of the plurality of previously diagnosed biometric images;
a second search module for searching for at least one previously diagnosed biometric image previously diagnosed by a predetermined diagnostician from the DB;
a communication module for transmitting the at least one similar biometric image and the at least one previously diagnosed biometric image to a terminal of the diagnostician of the diagnostician; and
a storage module for storing a disease diagnosis result of the diagnosis target biometric image received from the terminal of the terminal of the diagnostician in the DB, wherein
the terminal of the terminal of the diagnostician displays the diagnosis target biometric image, the at least one similar biometric image, and the at least one previously diagnosed biometric image.

2. The system according to claim 1, wherein the second search module searches for an image having characteristics similar to those of the diagnosis target biometric image among the biometric images previously diagnosed by the diagnostician from the DB.

3. The system according to claim 1, wherein the terminal of the terminal of the diagnostician displays the diagnosis target biometric image in a first region of a diagnosis UI including the first region, a second region, and a third region, displays the at least one similar biometric image in the second region, and displays the at least one previously diagnosed biometric image in the third region.

4. The system according to claim 3, wherein the diagnosis UI further includes a fourth region, and the terminal of the terminal of the diagnostician displays an image enlarging the diagnosis target biometric image, any one among the at least one similar biometric image, or any one among the at least one previously diagnosed biometric image in the fourth region.

5. The system according to claim 1, wherein the disease is cancer.

6. A biometric image diagnosis system comprising:
a search module for searching for at least one previously diagnosed biometric image having characteristics similar to those of a predetermined diagnosis target biometric image among biometric images previously diagnosed by a predetermined diagnostician from a DB that stores a plurality of previously diagnosed biometric images and diagnosis results of the plurality of previously diagnosed biometric images;
a communication module for transmitting the at least one similar biometric image and the at least one previously diagnosed biometric image to a terminal of the diagnostician; and
a storage module for storing a disease diagnosis result of the diagnosis target biometric image received from the terminal of the diagnostician in the DB, wherein
the terminal of the diagnostician displays the diagnosis target biometric image and the at least one previously diagnosed biometric image.

7. A terminal allowing a diagnostician to diagnose a disease through a biometric image, the terminal comprising:
a reception module for receiving at least one similar biometric image having characteristics similar to those of a predetermined diagnosis target biometric image, and at least one previously diagnosed biometric image previously diagnosed by the diagnostician from a biometric image diagnosis system;
a display module for displaying the diagnosis target biometric image, the at least one similar biometric image, and the at least one previously diagnosed biometric image; and
an annotation module for annotating a disease diagnosis result of the diagnosis target biometric image on the diagnosis target biometric image, wherein
the display module displays the diagnosis target biometric image in a first region of a diagnosis UI including the first region, a second region, and a third region, displays the at least one similar biometric image in the second region, and displays the at least one previously diagnosed biometric image in the third region.

8. A biometric image diagnosis method comprising the steps of:
searching for at least one similar biometric image having characteristics similar to those of a predetermined diagnosis target biometric image from a DB that stores a plurality of previously diagnosed biometric images and diagnosis results of the plurality of previously diagnosed biometric images, by a biometric image diagnosis system;
searching for at least one previously diagnosed biometric image previously diagnosed by a predetermined diagnostician from the DB, by the biometric image diagnosis system;
transmitting the at least one similar biometric image and the at least one previously diagnosed biometric image to a terminal of the diagnostician, by the biometric image diagnosis system; and
storing a disease diagnosis result of the diagnosis target biometric image received from the terminal of the diagnostician in the DB, by the biometric image diagnosis system, wherein
the terminal of the diagnostician displays the diagnosis target biometric image, the at least one similar biometric image, and the at least one previously diagnosed biometric image.

9. The method according to claim 8, wherein the step of searching for at least one previously diagnosed biometric image previously diagnosed by the diagnostician from the DB includes the step of searching for an image having characteristics similar to those of the diagnosis target biometric image among the biometric images previously diagnosed by a user from the DB.

10. A biometric image diagnosis method comprising the steps of:
searching for at least one previously diagnosed biometric image having characteristics similar to those of a diagnosis target biometric image among biometric images previously diagnosed by a predetermined diagnostician from a DB that stores a plurality of previously diagnosed biometric images and diagnosis results of the plurality of previously diagnosed biometric images, by a biometric image diagnosis system;
transmitting the at least one previously diagnosed biometric image to a terminal of the diagnostician, by the biometric image diagnosis system; and
storing a disease diagnosis result of the diagnosis target biometric image received from the terminal of the diagnostician in the DB, by the biometric image diagnosis system, wherein
the terminal of the diagnostician displays the diagnosis target biometric image and the at least one previously diagnosed biometric image.

11. A biometric image diagnosis method executed in a terminal allowing a diagnostician to diagnose a disease through a biometric image, the method comprising the steps of:
receiving at least one similar biometric image having characteristics similar to those of a predetermined diagnosis target biometric image, and at least one previously diagnosed biometric image previously diagnosed by the diagnostician from a biometric image diagnosis system, by the terminal;
displaying the diagnosis target biometric image, the at least one similar biometric image, and the at least one previously diagnosed biometric image, by the terminal; and
annotating a disease diagnosis result of the diagnosis target biometric image on the diagnosis target biometric image, wherein
the step of displaying the diagnosis target biometric image, the at least one similar biometric image, and the at least one previously diagnosed biometric image includes the steps of:
displaying the diagnosis target biometric image in a first region of a diagnosis UI including the first region, a second region, and a third region;
displaying the at least one similar biometric image in the second region; and
displaying the at least one previously diagnosed biometric image in the third region.

12. A computer program installed in a data processing apparatus and recorded in a medium for executing the method disclosed in any one of claims 8, 10 and 11.
